# FASCICULE DE BREVET EUROPEEN

(11) **EP 1 415 603 B1**
(45) Date de publication et mention de la délivrance du brevet: **23.04.2008**
(21) Numéro de dépôt: 03025177.1
(22) Date de dépôt: 03.06.1998
(51) Int. Cl.: A61B 17/70, A61B 17/88

(54) **Dispositif d'ostéosynthèse vertébrale pluridirectionnelle et modulable, à encombrement réduit**
Modulare, platzsparende und multidirektionelle Osteosynthesevorrichtung für die Wirbelsäule
Multidirectional adaptable vertebral osteosynthesis device with reduced space requirement

(30) Priorité: 03.06.1997 IE 970411
(43) Date de publication de la demande: 06.05.2004
(62) Demande divisionnaire de: 98929473.1
(73) Titulaire: Médicréa Technologies, 17000 La Rochelle (FR)
(72) Inventeur: Bernard Villaret, 17220 Croix-Chapeau (FR); Jean Taylor, 06400 Cannes (FR)
(74) Mandataire: Jeannet, Olivier

(56) Documents cités:
- US-A- 5 628 740

## Description

La présente invention a pour objet un dispositif d'ostéosynthèse rachidienne, en particulier dorso-lombaire.

Plus précisément, l'invention vise un dispositif du type comprenant au moins deux éléments d'ancrage osseux dans des structures osseuses du rachis, un organe de liaison longitudinale entre les éléments d'ancrage osseux, et des étriers de connexion entre les éléments d'ancrage osseux et les organes de liaison entre les vis ; chaque élément d'ancrage osseux comporte une prise d'ancrage osseux, une tête de préhension par un outil de vissage, un axe fileté prolongeant la tête de préhension et un élément de serrage pouvant être monté sur cet axe pour bloquer ensemble le connecteur, l'organe de liaison longitudinale et l'élément d'ancrage osseux correspondant.

L'ostéosynthèse plurivertébrale, notamment dorso-lombaire, combine le recours à des vis ou des crochets, reliés entre eux par des plaques ou des tiges.

L'utilisation de plaques comportant des évidements adaptés, autorise un certain débattement des vis et le glissement de ces dernières le long d'un axe. Ceci est utile lors d'implantation de vis ayant une divergence dans le plan sagittal.

L'utilisation d'organes de liaison longitudinale tels que par exemple des tiges permet, en outre, de faire coulisser les éléments d'ancrage osseux, par exemple des vis, le long de l'axe principal de l'organe de liaison longitudinale, de ramener dans le même axe antéro-postérieur, des vis divergentes dans le plan horizontal, et ce, grâce à des effets de dérotation imprimés aux tiges autour d'un axe apicocaudal, c'est-à-dire dans le plan horizontal.

Cependant, le cintrage de la tige qu'impose cette manoeuvre, doit être effectué entre deux segments vertébraux suffisamment distants. D'autre part, un ou plusieurs cintrages successifs ne s'effectuent que dans le même plan frontal. On aboutit ainsi à transposer une déformation dans un autre plan, orthogonal au premier.

L'ajustement du couple vis pédiculaires-tige, peut conduire à des sollicitations très importantes du système avant son verrouillage définitif. A cet effet, des instruments spécifiques ont été imaginés.

On a également mis au point des vis pédiculaires dont l'axe fileté est prolongé en arrière, afin de pouvoir guider segment par segment la descente de la tige jusqu'à la base d'implantation vertébrale de la vis.

L'autre intérêt de ce type d'implant pédiculaire rallongé, est d'autoriser indifféremment le recours à une plaque ou à une tige.

Il est des déformations dont le rayon de courbure peut être très court, uni ou bisegmentaire, mais cependant combiné dans les trois plans, sagittal, horizontal et frontal. Le simple cintrage monoplanaire d'une tige, amenée progressivement à quai, ou en effectuant un mouvement global de dérotation, n'est alors plus adapté.

En effet, la réduction par rotation de la tige en cas de cintrage dans deux plans est prohibée par les lois mécaniques.

La réduction d'une déformation à grand rayon, dans ces conditions, est tri-planaire, mais en aucun cas séquentielle, et encore moins sélective.

Ces déformations courtes, partiellement réductibles, doivent être considérées segment par segment, et surtout plan par plan, avant d'envisager toute manoeuvre de réduction, notamment partielle.

Une vertèbre isolément décalée dans les plans, frontal, sagittal et horizontal, doit être mise en condition de subir une réduction dans un seul plan si cela est nécessaire, voire même en vue d'être solidarisée telle qu'elle au segment adjacent, sans autre contrainte que celle induite par la neutralisation.

Pour résoudre cette équation, des vis pédiculaires munies d'un système à "rotule", ont été imaginées et mises au point.

Ainsi, la tête d'une vis peut être coiffée d'un élément en forme de "U", ainsi dénommé "tulipe", qui acquiert une mobilité autour de l'axe principal de la vis.

Le débattement obtenu autorise, dans certaines limites, de se soustraire aux conséquences d'un décalage angulaire dans le plan horizontal et/ou frontal de l'alignement pédiculaire.

Dans ces conditions, le cintrage de la tige n'est plus un artifice servant à aligner tant bien que mal un montage mal axé frontalement.

Le chirurgien se trouvé libéré de cette énorme contrainte et peut implanter les vis pédiculaires dans l'axe qu'impose la topographie de la vertèbre pathologique.

La statique vertébrale sagittale régionale est respectée grâce à un cintrage monoplanaire, visant à la restitution de l'équilibre sagittal. Différentes solutions mécaniques sont proposées, par, notamment, emboîtements successifs d'éléments aboutissant à la solidarisation du trinôme, vis, rotule, tige.

Des évidements géométriquement complexes, et l'emboîtement d'une série d'éléments, permettent de reproduire les avantages de l'élément vis-tulipe à rotule, précédemment décrit.

Malgré le progrès considérable que représente cette alternative, il convient d'en faire une analyse critique, qui se résumera en trois points.
1. Les vis en U polyaxiales, ne permettent pas d'une part l'interchangeabilité tige-plaque, sinon au prix d'un démontage relevant de la "poupée russe".
   Par ailleurs, la réduction d'un antélisthésis oblige à recourir à des vis munies d'un U dont les bras sont prolongés en arrière, ce au prix d'un encombrement beaucoup plus important. Enfin, afin de ne pas solliciter les éléments de serrage lors de la manoeuvre de traction, le recours à un instrument spécifique de réduction, est recommandé, mais conduit à solliciter le pédicule en traction ; autant d'efforts qui produisent une fragilisation préalable.
2. L'usage d'entretoises successives, peut s'avérer fastidieux, multipliant les manoeuvres.
   Le caractère mécaniquement fiable du blocage suppose un ajustement parfait, mais aléatoire dans un champ opératoire (contrainte de la voie d'abord, interposition de tissus, mauvais contrôle visuel...) du niveau d'enfoncement de l'implant.
   L'absence de verrouillage rotationnel entre la partie d'ancrage et la rotule polyaxiale rend en outre le démontage difficile, parfois impossible.
3. Les instruments spécifiques auxquels il faut recourir, sont autant d'aléas qui grèvent le temps opératoire, imposent l'apprentissage des ancillaires médicaux, et enfin alourdissent la maintenance.

Le document US 5,628,740 décrit un axe fileté équipé d'ergots venant coopérer avec des rainures que comprend la cavité aménagée intérieurement dans la tête de vis pour recevoir la partie sphérique de l'axe.

Un dispositif selon l'invention comprend au moins deux éléments d'ancrage osseux dans des corps de la structure osseuse du rachis respectifs, au moins un organe de liaison longitudinale entre les éléments d'ancrage osseux et des étriers de connexion, chaque élément d'ancrage osseux comportant une tête de préhension par un outil de vissage, un axe fileté prolongeant la tête de préhension, et un élément de serrage pouvant être mis en place sur cet axe pour bloquer ensemble l'organe de liaison longitudinale et l'élément d'ancrage osseux correspondant, l'axe fileté étant pourvu d'une rotule terminale d'articulation dans un logement d'une calotte sphérique de la tête de préhension, permettant une orientation pluridirectionnelle de l'axe et un positionnement de l'organe de liaison longitudinale adaptés à la configuration du segment vertébral recevant les éléments d'ancrage osseux ; le dispositif est caractérisé en ce que l'axe fileté et chaque étrier de connexion sont munis de moyens de blocage venant coopérer après introduction de l'axe fileté dans un trou correspondant de passage à travers l'étrier de connexion, pour bloquer en rotation l'axe et sa rotule par rapport à l'étrier de connexion.

Un autre dispositif selon l'invention comprend au moins deux éléments d'ancrage osseux dans des corps de la structure osseuse du rachis respectifs, au moins un organe de liaison longitudinale entre les éléments d'ancrage osseux et des étriers de connexion, chaque élément d'ancrage osseux comportant une tête de préhension par un outil de vissage, un axe fileté prolongeant la tête de préhension, et un élément de serrage pouvant être mis en place sur cet axe pour bloquer ensemble l'organe de liaison longitudinale et l'élément d'ancrage osseux correspondant, l'axe fileté étant pourvu d'une rotule terminale d'articulation dans un logement d'une calotte sphérique de la tête de préhension, permettant une orientation pluridirectionnelle de l'axe et un positionnement de l'organe de liaison longitudinale adaptés à la configuration du segment vertébral recevant les éléments d'ancrage osseux ; le dispositif est caractérisé en ce que l'axe fileté et l'organe de liaison longitudinale sont munis de moyens de blocage venant coopérer après introduction de l'axe fileté dans un trou correspondant de passage à travers l'organe de liaison longitudinale, pour bloquer en rotation l'axe et sa rotule par rapport à l'organe de liaison longitudinale.

Avantageusement, lesdits moyens comprennent au moins une géométrie d'arrêt rotationnel formée entre la rotule et l'extrémité contiguë de l'axe fileté, et une seconde géométrie d'arrêt rotationnel ménagée sur le bord intérieur du trou d'un étrier connecteur, cette seconde géométrie étant adaptée pour venir s'appliquer sur la première géométrie après coulissement de l'étrier connecteur sur l'axe fileté.

D'autres particularités et avantages de l'invention apparaîtront au cours de la description qui va suivre, faite en référence aux dessins annexés qui en illustrent deux formes de réalisation à titre d'exemples non limitatifs.

La figure 1 est une vue en perspective partielle avant assemblage, à échelle agrandie, d'une première forme de réalisation du dispositif d'ostéosynthèse rachidienne selon l'invention.

La figure 2 est une vue en perspective partielle du dispositif de la figure 1, montrant à échelle agrandie, un élément d'ancrage osseux à double filetage et un étrier correspondant de connexion avec une tige vertébrale non représentée, cet élément d'ancrage osseux pouvant être notamment une vis ou un crochet.

La figure 3 est une vue en perspective à échelle agrandie du dispositif des figures 1 et 2 assemblées et mises en place sur un segment vertébral.

La figure 4 est une vue en élévation antérolatérale d'un segment dorso-lombaire avec un dispositif d'ostéosynthèse selon l'invention en cours de montage, certains des étriers connecteurs équipant une tige vertébrale étant enfilés sur les axes filetés des éléments d'ancrage osseux correspondants préalablement ancrés dans les structures osseuses vertébrales.

La figure 5 est une vue postérieure du segment dorso-lombaire de la figure 4 et du dispositif correspondant installé.

La figure 6 est une vue antérolatérale du dispositif de la figure 5, montrant la lordose lombaire assurée par le cintrage de la tige vertébrale.

La figure 7 est une vue en plan d'un élément d'ancrage osseux monobloc, sans rotule, pouvant équiper le dispositif d'ostéosynthèse selon l'invention.

La figure 8 est une vue en élévation postérieure d'un dispositif à plaque de liaison entre les éléments d'ancrage osseux monté sur un segment dorsolombaire.

La figure 9 est une vue en élévation dans un plan sagittal du dispositif à plaque de la figure 8, comportant un élément d'ancrage osseux tel que celui de la figure 6.

La figure 10 est une vue en élévation et coupe partielles à échelle agrandie de l'assemblage d'un élément d'ancrage osseux, d'un étrier connecteur et d'un élément de serrage selon la réalisation des figures 1 à 4, assurant un rappel de l'élément d'ancrage osseux dans l'axe de l'élément de serrage.

La figure 11 est une vue schématique en élévation, à échelle réduite par rapport à la figure 10, de l'ensemble du dispositif correspondant, illustrant le rappel angulaire de l'élément d'ancrage osseux dans l'axe de l'élément de serrage et de la tige filetée lors du serrage.

La figure 12 est une vue partielle analogue à la figure 10 d'une variante de réalisation du dispositif, modifiée de manière à ne pratiquement pas assurer de rappel angulaire appréciable de l'élément d'ancrage osseux lors du serrage.

La figure 13 est une vue en perspective à échelle agrandie, d'un second mode de réalisation de l'étrier connecteur de la figure 2.

La figure 14 est une vue en élévation à échelle agrandie d'un second mode de réalisation d'un élément d'ancrage osseux du dispositif.

La figure 15 est une vue en coupe partielle et élévation d'un mode de réalisation d'un système de liaison transversale entre deux éléments d'ancrage osseux, pouvant équiper le dispositif des figures 1 à 14.

La figure 16 est une vue de dessus du système de liaison transversale de la figure 15.

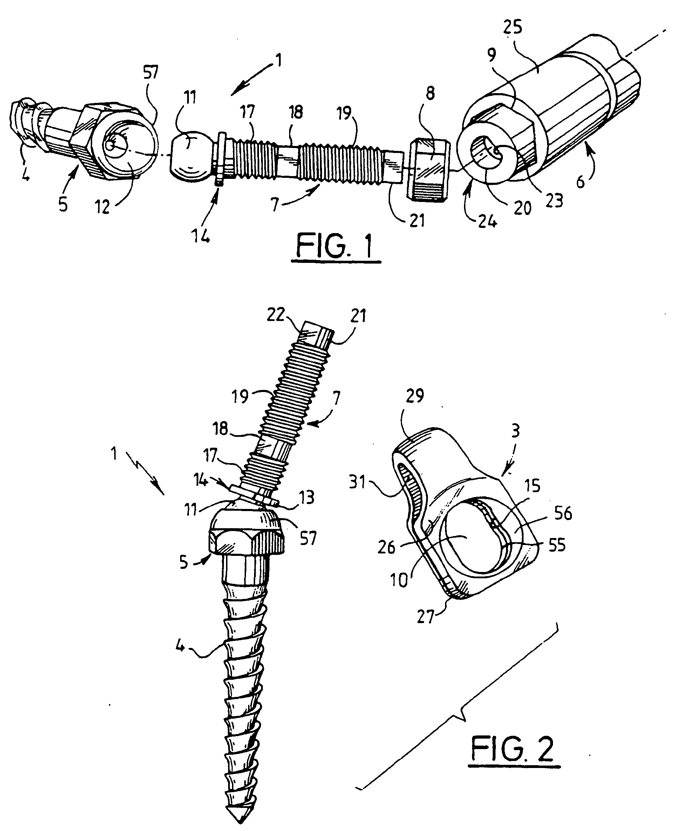

Le dispositif d'ostéosynthèse rachidienne illustré aux figures 1 à 6 comprend plusieurs éléments d'ancrage osseux, constitués dans l'exemple décrit par des éléments d'ancrage osseux 1 dans des vertèbres respectives, un organe de liaison longitudinale entre les éléments d'ancrage osseux 1 constitué par une tige vertébrale 2, et des étriers 3 de connexion entre les éléments d'ancrage osseux 1 et les tiges vertébrales 2, à raison d'un étrier 3 par élément d'ancrage osseux 1.

Chaque élément 1 comporte une tige filetée conique 4 d'ancrage osseux, une tête 5 de préhension par un outil de vissage 6, un axe fileté mécanique 7 prolongeant la tête 5. Le dispositif est complété par un écrou 8 pouvant être vissé sur l'axe fileté 7 pour bloquer ensemble l'étrier connecteur 3, la tige vertébrale 2 et l'élément d'ancrage osseux correspondant 1.

La tête de préhension 5 comporte une forme pouvant coopérer avec l'outil de vissage 6, par exemple un contour hexagonal comme représenté, adapté pour coopérer avec une empreinte hexagonale femelle 9 de l'outil 6.

L'axe 7 est pourvu d'une rotule terminale 11 d'articulation dans un logement hémisphérique 12 de la tête 5, dans lequel cette rotule 11 peut être maintenue par assemblage divers et notamment sertissage, soudure, etc. Le logement 12 sensiblement hémisphérique permet à la rotule 11 de tourner et d'être mobilisée dans tous les plans, autorisant ainsi une orientation pluridirectionnelle de l'axe fileté 7.

Ce dernier et l'étrier 3 de connexion sont munis de moyens pour bloquer en rotation l'axe 7 et sa rotule 11, pendant le serrage ou le desserrage de l'écrou 8, après introduction de l'axe 7 dans un trou correspondant 10 de passage à travers l'étrier de connexion 3. Dans la réalisation représentée, ces moyens comprennent au moins une géométrie d'arrêt rotationnel mâle 13 formée sur une collerette 14 agencée entre la rotule 11 et l'extrémité contiguë de l'axe 7 et au moins une seconde géométrie rotationnelle femelle illustrée par un méplat 15 ménagé sur le bord intérieur du trou 10 de l'étrier 3. Ce second méplat 15 est adapté pour pouvoir venir s'appliquer sur le premier méplat 13 après coulissement de l'étrier 3 sur l'axe fileté 7.

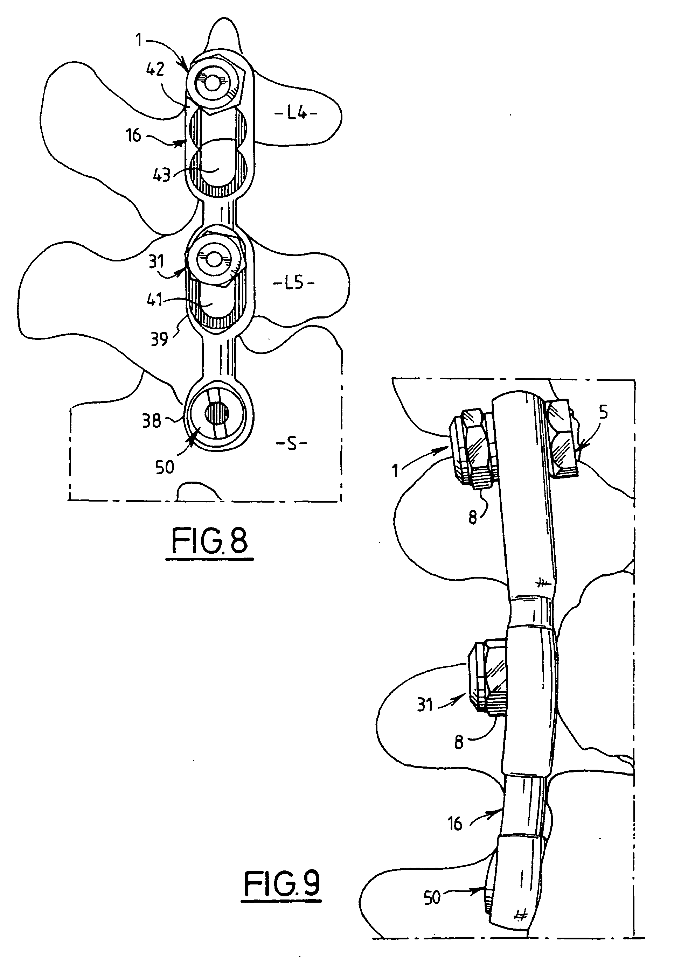

De préférence la collerette 14 présente ainsi deux géométries d'arrêt rotationnel 13 diamétralement opposées, l'une seulement de ces géométries 13 étant visibles aux dessins. La collerette 14 ainsi munie des deux géométries 13 peut venir s'ajuster dans l'étrier connecteur correspondant 3 si le montage est utilisé avec une tige vertébrale 2, ou dans une plaque 16 ayant des géométries d'arrêt rotationnel (bords des trous 38, 41, 43 aux Fig. 8 et 9) similaires 13 (figures 8 et 9) si l'on utilise une plaque 16 à la place de la tige 2 comme organe de liaison longitudinale entre les vis 1.

Au-delà de la collerette 14, l'axe 7 comporte une première portion filetée cylindrique 17, un rétrécissement 18 constituant une zone d'amorce de rupture, une seconde portion filetée cylindrique 19 prolongée par une partie terminale 21 lisse et constituant une forme mâle de profil approprié, par exemple en demi-lune avec une géométrie d'arrêt rotationnel, ci-après le méplat 22 (figure 2). Cette forme mâle 21 est adaptée pour pouvoir coopérer avec une forme femelle 20 complémentaire de l'outil 6, ménagée dans l'extrémité d'un manchon 24 monté coulissant axialement à l'intérieur d'une douille 25, à l'extrémité de laquelle est agencée l'empreinte femelle hexagonale 9 (figure 1).

La zone d'amorce de rupture 18 a de préférence une géométrie d'arrêt rotationnel identique au méplat 22. Ce agencement permet de bloquer en rotation la rotule 11, lors d'une opération de reprise de l'implant, à l'aide de l'outil 6.

L'emboîtement de la forme mâle 21 avec sa géométrie d'arrêt rotationnel pouvant être un méplat 22 dans la forme femelle conjuguée 20 avec application des méplats 22 et 23 l'un contre l'autre, permet de bloquer l'axe fileté 7 en rotation pendant le vissage de l'écrou 8 sur les portions filetées 19 et 17 de l'axe 7.

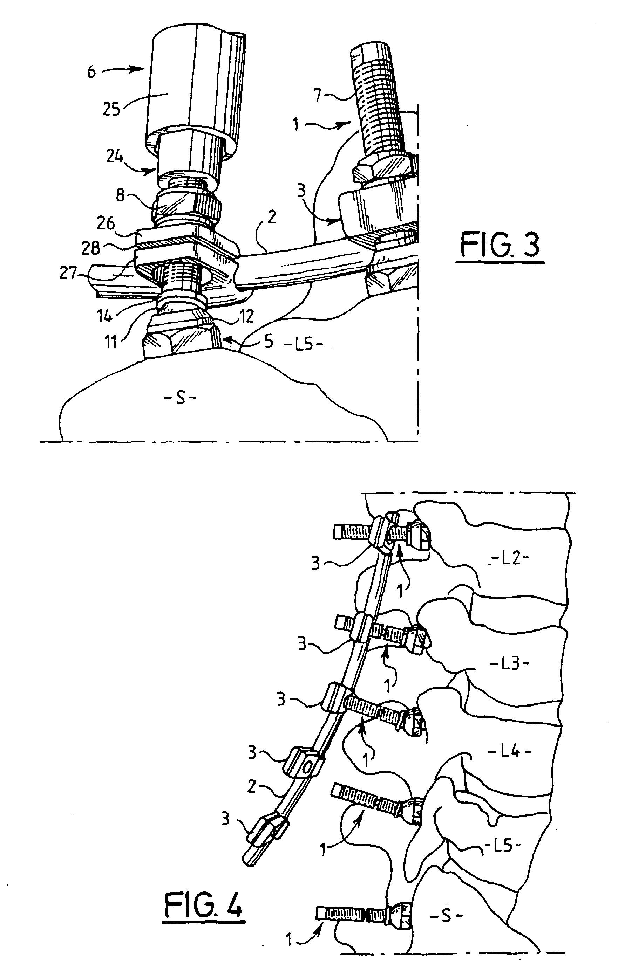

Par ailleurs une fois le montage terminé, c'est au niveau du rétrécissement 18 que s'opère la cassure de l'axe 7 en deux parties, afin de retirer la portion filetée 19. Ainsi, seule la portion filetée 17 fait partie intégrante du montage définitif, la seconde portion 19 ayant pour fonction uniquement de guider la descente de l'écrou 8 jusqu'à l'étrier 3 (figure 3). Durant la descente de l'écrou 8, l'emboîtement des méplats mâle 22 et femelle 23 du manchon 24 assure le blocage en rotation de la rotule 11 dans son logement 12,

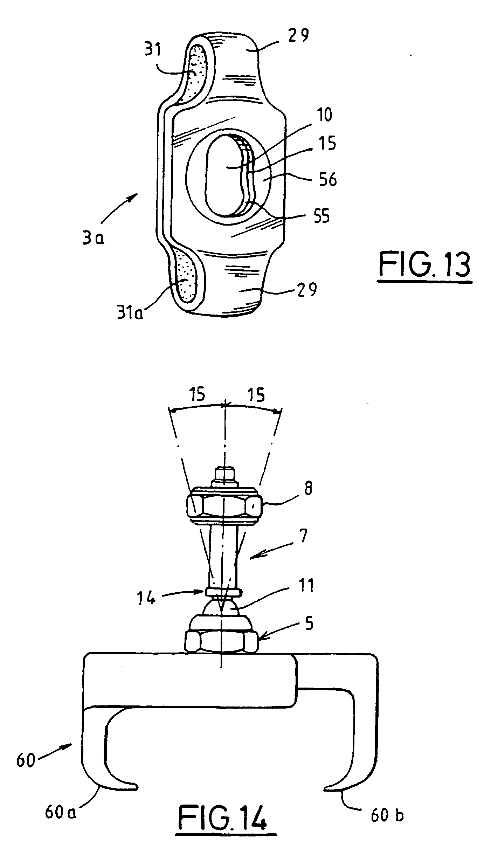

L'étrier connecteur 3 est constitué de deux branches 26, 27 repliées l'une sur l'autre et séparées par une fente longitudinale 28, le trou 10 de passage de l'axe 7 étant ainsi formé dans les branches 26, 27 de part et d'autre de la fente 28. Les deux branches 26, 27 sont reliées par un ou deux raccords arrondis 29 qui délimite un ou deux logements cylindriques 31 dans lequel peuvent être introduites une ou deux tiges cylindriques 2 (figure 13).

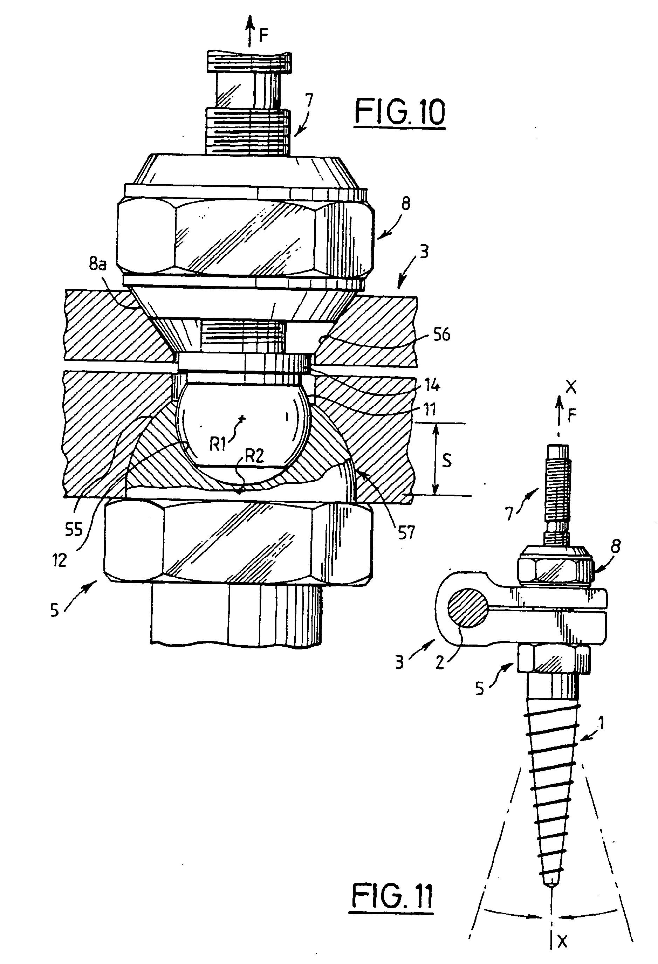

Les figures 10 et 11 illustrent plus en détail la réalisation du dispositif qui vient d'être décrit en référence aux figures 1 à 3.

En effet, elles montrent que la sphère ou rotule 11 de l'élément d'ancrage osseux 1 et la calotte sphérique 57 présentent des centres de rotation respectifs R1 et R2, distincts et séparés par un écartement S. La surface de la calotte 57 de la tête 5 est hémisphérique et interrompue dans sa zone polaire pour recevoir la rotule 11, et la surface sphérique 55 associée de l'étrier 3, de même rayon de courbure que la surface de la calotte hémisphérique 57, recouvre complètement cette dernière.

L'appui pris sur la partie supérieure de la tête de préhension 5 assure au système étrier connecteur 3 et élément d'ancrage osseux 1 une fonction de rappel de ce dernier dans l'axe XX de l'écrou de serrage 8 et de la tige filetée 7 lors de la manoeuvre de serrage par l'élément 8. En effet, au moment de cette manoeuvre, l'élément 8 (écrou par exemple) dont la jupe 8a vient s'appliquer sur la paroi conique 56 de l'évidement de l'écrou 8, produit une force de traction F (figure 10), qui détermine un couple C (figure 11) de rappel de l'élément d'ancrage osseux 4 vers l'axe longitudinal XX de l'élément de serrage 8 et de l'axe fileté 7 par une force orthogonale à cet axe.

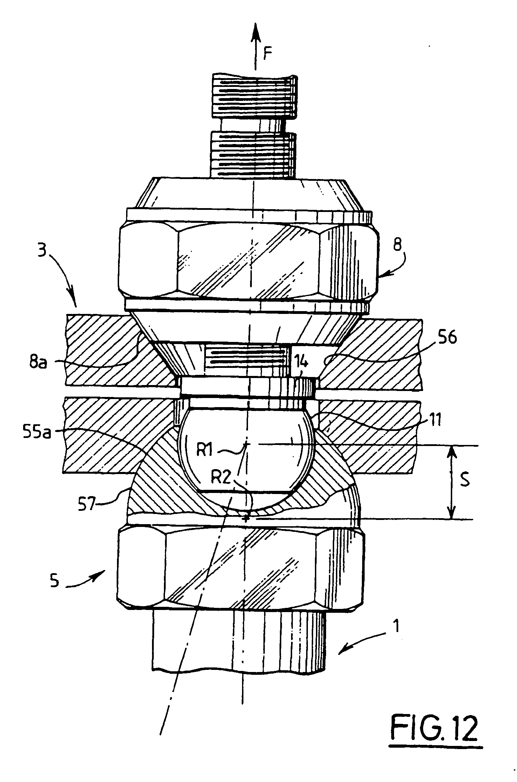

Dans la forme de réalisation illustrée à la figure 12, la surface sphérique 55a ne recouvre que partiellement la surface sphérique de la calotte 57 car la portée sphérique 55a est interrompue nettement avant l'équateur de la calotte 57. De ce fait, la force de traction F produite par le serrage de l'écrou 8, immobilise l'étrier connecteur 3 par contact surfacique, tout en conservant l'orientation de l'élément d'ancrage osseux 1.

Cette possibilité d'agir avec des connecteurs différents pouvant faire varier le réalignement, autorise une planification des corrections, sans recours à des outils complémentaires.

La figure 13 illustre une forme de réalisation de l'étrier connecteur 3a dans laquelle celui-ci comporte, de part et d'autre du trou 10, deux raccords arrondis 29, 29a délimitant deux logements respectifs 31, 31a adaptés pour recevoir des organes de liaison longitudinale tels que des tiges vertébrales.

La figure 14 illustre un second mode de réalisation de l'élément d'ancrage osseux, ici constitué par un crochet lamaire 60 remplaçant la tige filetée 4 de la réalisation précédente, le reste du dispositif étant par ailleurs similaire à celui des figures 1 et 2, notamment la tête 5 de préhension par un outil de vissage 6 et l'axe fileté 7. Le crochet lamaire 6 est constitué de manière connue en soi par deux pinces 60a, 60b à extrémité recourbée et d'écartement relatif réglable.

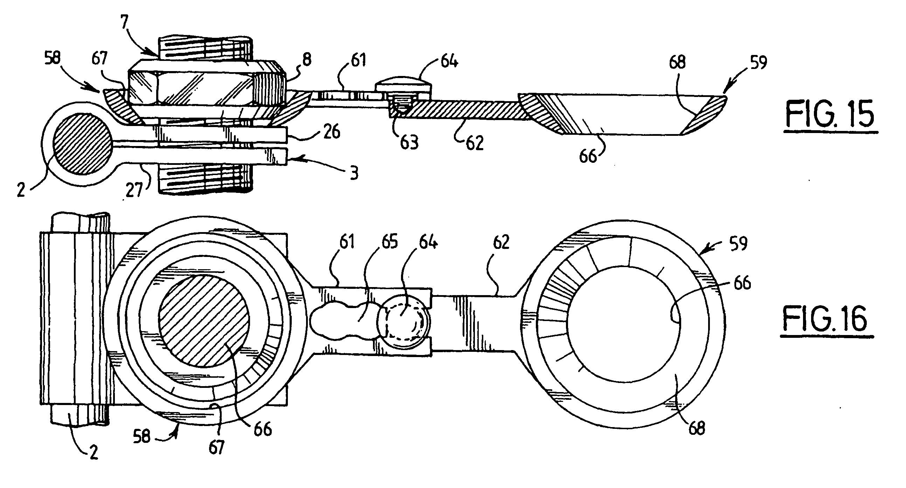

Les figures 15 et 16 illustrent un mode de réalisation possible d'un système de liaison transversale entre des éléments d'ancrage osseux (1 ou 31 ou 60). Ce système de liaison est formé par une paire de cuvettes 58, 59 évasées, dont le fond est percé d'une ouverture 66 de passage de l'axe fileté 7. Chaque cuvette 58, 59 est réalisée monobloc avec une patte transversale respective 61, 62, la position relative, et donc l'écartement entre les cuvettes, étant réglable. Ce réglage peut être obtenu par exemple au moyen d'un ensemble vis-écrou 63, 64 traversant une lumière allongée 65 d'une patte 61 et un trou taraudé de la deuxième patte 62. Chaque cuvette 58, 59 est interposée entre un étrier connecteur 3 (ou 3a) et un élément de serrage correspondant 8, qui vient se visser dans la cuvette en prenant appui sur sa paroi conique 67, 68 par sa jupe conique 8a.

L'orientabilité de l'élément d'ancrage osseux 1 par rapport à l'axe XX, avec rappel (figures 10, 11) ou sans rappel angulaire (figure 12), peut aussi être obtenue avec un évidement géométriquement complexe similaire réalisé dans une plaque telle que 16 (figures 8 et 9, orifice 41, 43).

Une fois l'élément d'ancrage 4 préalablement appliqué à la structure d'une vertèbre, par exemple lombaire, on oriente l'axe 7 vers le connecteur 3 correspondant, préalablement monté sur une tige vertébrale 2. Une fois cette introduction effectuée, l'outil 6 permet de bloquer l'axe 7 en rotation grâce au manchon 24 tandis que la douille extérieure 25 permet de visser l'élément de serrage 8 jusqu'à sa position de blocage de l'ensemble, la ou les géométries d'arrêt rotationnel 13 de la collerette 14 étant appliquées sur la ou les géométries d'arrêt rotationnel correspondantes 15 de l'étrier 3.

La figure 4 illustre une manoeuvre de réduction. La tige vertébrale 2 a été cintrée dans le plan sagittal pour reproduire la courbure de la lordose que l'on souhaite rétablir. Les étriers connecteurs 3 sont enfilés sur la tige 2 qui, par l'intermédiaire des étriers 3, est guidée pas à pas, mais sans effort, car la rotule 11 de chaque élément d'ancrage osseux 1 permet de diriger l'axe fileté extra pédiculaire 7 vers l'étrier 3, avant d'entreprendre d'amener la tige 2 au contact de la colonne vertébrale- à savoir dans l'exemple représenté un segment dorso-lombaire : sacrum S et vertèbres lombaires L5, L4, L3, L2. La descente de l'étrier 3 le long de l'axe fileté 7 constituant la portion mobile de l'implant s'effectue grâce à l'élément de serrage 8 (écrou), à l'aide de la clé constituée par l'outil 6, empêchant la rotule 11 de tourner sur elle-même comme expliqué précédemment. L'étrier 3 vient par sa face inférieure rejoindre la collerette 14 convenablement orientée, les deux géométries d'arrêt rotationnel 22 (méplats) 23 se rejoignent, assurant ainsi le blocage de la rotule 11. En effet, une fois en regard du méplat 15 de l'étrier 3, la collerette 14 ne peut plus tourner autour de son axe. Lorsque les deux géométries d'arrêt rotationnel mâle 22 et femelle 15 sont en face l'un de l'autre, la rotule 11 se bloque d'elle-même. L'implant est devenu mono-axial.

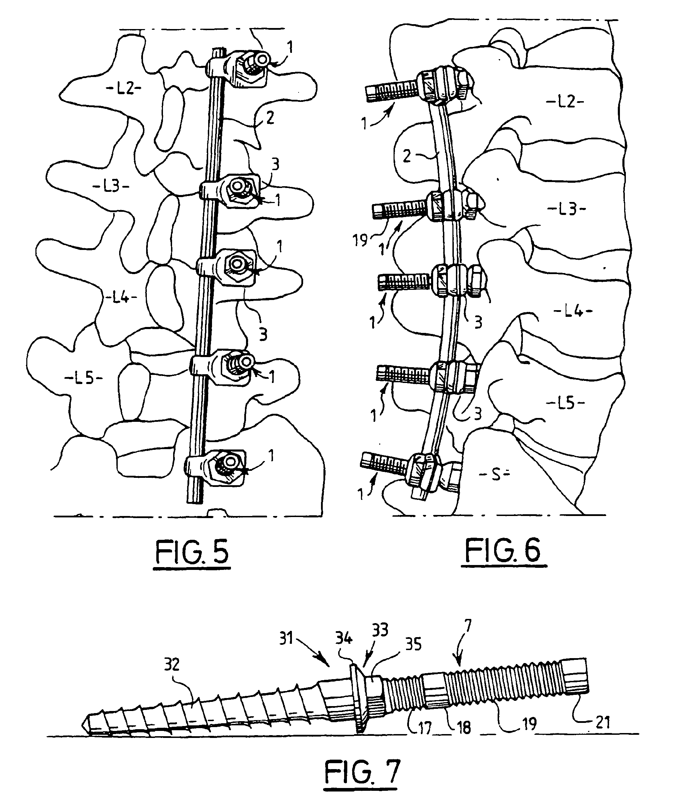

Dans le montage lombosacré illustré à la figure 6, intéressant le sacrum S et les quatre premières vertèbres lombaires, on voit que la lordose physiologique a été rétablie grâce à la courbure de la tige 2 dans le plan sagittal, les portions extra-pédiculaires constituées par les axes 7 étant corrélativement orientées pour s'adapter à ce cintrage. Une fois le montage verrouillé, la portion postérieure 19 de chaque axe fileté 7 est sectionnée aisément, grâce à la réduction de section formée par la zone d'amorce de rupture 18. Les radios postopératoires de patients présentant une scoliose lombaire permettent de vérifier que grâce au dispositif d'ostéosynthèse selon l'invention, les implants pédiculaires 1, vus de face, ne sont pas dans le même plan, et que la lordose lombaire, (de profil) a été rétablie de manière très satisfaisante, avec notamment réapparition d'une asymétrie discale physiologique, indispensable pour créer des conditions anatomiquement correctes.

La figure 7 illustre un second élément d'ancrage osseux 31 (une vis dans cet exemple) pouvant être mis en oeuvre dans le dispositif selon l'invention, lorsque celui-ci comprend une plaque 16 (figures 8 et 9) ou des étriers connecteurs 3.

La plaque 16 présente en regard du sacrum S une partie terminale à un trou circulaire de passage d'un élément d'ancrage osseux unique 31, puis au niveau de L5 une seconde portion 39 allongée dans laquelle est formé un trou oblong 41 permettant d'ajuster corrélativement la position d'un élément d'ancrage osseux 31 entre deux positions ; enfin la plaque 16 comporte une troisième partie 42 de forme allongée dans laquelle est ménagé un passage oblong 43 délimitant trois positions possibles pour l'élément d'ancrage osseux 1 selon l'ajustement nécessaire, grâce à trois échancrures ménagées sur les bords du passage 43.

La plaque 16, destinée à trois segments ou étages rachidiens S, L5, L4, par exemple, peut être remplacée par une plaque adaptée à un nombre différent d'étages. Par exemple dans le montage à trois étages des figures 8 et 9, seul un élément d'ancrage osseux est polyaxial, donc à rotule 11, les autres éléments d'ancrage osseux 31 étant monoaxiaux. Chaque trou (41 ...) de la plaque 16 peut avoir le même profil que le trou 10 de l'étrier connecteur 3 pour le passage de l'élément d'ancrage osseux (Fig. 10). Ce profil permet d'assurer une fonction de rappel de l'élément osseux vers l'axe longitudinal de l'élément de serrage et de l'axe fileté 7 par une force orthogonale à cet axe. La collerette 34 située dans le prolongement de la portion intrapédiculaire de l'élément d'ancrage osseux 1, est fixe (figures 8 et 9). Elle peut utilement assurer un bon appui contre la vertèbre par un effet dit "console", alors qu'un élément d'ancrage osseux 1 peut utilement servir à réduire segmentairement un angle entre deux structures osseuses du rachis contiguës.

La vis polyaxiale 1 est laissée mobile au début de la mise en place de l'élément de serrage 8 le long de l'axe fileté 7. Ensuite le manchon 24 avec sa forme en demi-lune 23 vient bloquer la rotule 11. Par un mouvement ajusté, on vient ainsi positionner l'élément d'ancrage osseux 1 dans l'un des trois orifices du trou oblong 43. Le précintrage de la plaque 16 permet à la vertèbre L4 de se repositionner en lordose par rapport à la vertèbre sous-jacente, sans compromettre le verrouillage du couple plaque 16 - élément d'ancrage osseux 1, grâce à la tolérance de la rotule 11.

Il est possible d'utiliser une plaque pour deux structures osseuses du rachis lombaires seulement. Le précintrage de cette plaque permet de ramener la vertèbre en bascule postérieure et donc de recréer une asymétrie discale physiologique, dans le cadre notamment du traitement chirurgical d'une pathologie dite de "dos plat".

Outre les avantages techniques précédemment mentionnés, le dispositif d'ostéosynthèse rachidienne selon l'invention présente les avantages suivants :
- guidage de l'élément d'ancrage osseux 1, 31 par un instrument 6 normo-axant instantanément la partie pédiculaire 4, 32 de l'élément d'ancrage osseux 1 et son prolongement multiaxial 7.
- Possibilité de réduction monoplanaire ou combinée dans les trois plans.
- Affranchissement de certaines séquences opératoires.
- Réduction vertébrale par traction antéro-postérieure à l'aide de l'élément d'ancrage osseux, directement sans instrument complémentaire.
- Maîtrise de l'orientabilité du système susceptible d'être conservée ou neutralisée indifféremment en fonction des exigences per-opératoires, grâce aux caractéristiques dimensionnelles et fonctionnelles des étriers connecteurs 3 (combinaison de la portée sphérique 55 ou 55a avec la calotte sphérique 57).

## Revendications

1. Dispositif d'ostéosynthèse rachidienne, comprenant au moins deux éléments d'ancrage osseux (1, 60) dans des corps de la structure osseuse du rachis respectifs (S, L5), au moins un organe (2) de liaison longitudinale entre les éléments d'ancrage osseux et des étriers de connexion (3, 3a), chaque élément d'ancrage osseux comportant une tête (5) de préhension par un outil (6) de vissage, un axe fileté (7) prolongeant la tête de préhension, et un élément de serrage (8) pouvant être mis en place sur cet axe pour bloquer ensemble l'organe (2) de liaison longitudinale et l'élément d'ancrage osseux (1, 60) correspondant, l'axe fileté (7) étant pourvu d'une rotule terminale (11) d'articulation dans un logement (12) d'une calotte sphérique (57) de la tête (5) de préhension, permettant une orientation pluridirectionnelle de l'axe (7) et un positionnement de l'organe (2) de liaison longitudinale adaptés à la configuration du segment vertébral (S, L5, ... L2) recevant les éléments d'ancrage osseux, **caractérisé en ce que** l'axe fileté (7) et chaque étrier de connexion (3, 3a) sont munis de moyens de blocage (13, 14 ; 15) venant coopérer après introduction de l'axe fileté (7) dans un trou correspondant (10) de passage à travers l'étrier de connexion (3, 3a), pour bloquer en rotation l'axe (7) et sa rotule (11) par rapport à l'étrier de connexion (3, 3a).

2. Dispositif selon la revendication 1, **caractérisé en ce que** l'organe de liaison longitudinale entre les éléments d'ancrage osseux (1) est une tige vertébrale (2) traversant les étriers (3, 3a) de connexion aux éléments d'ancrage osseux, et **en ce que** lesdits moyens de blocage comprennent au moins une géométrie d'arrêt rotationnel, de préférence deux, à savoir une première géométrie d'arrêt rotationnel (13) formée sur une collerette (14) agencée entre la rotule et l'extrémité contiguë de l'axe fileté (7), et une seconde géométrie d'arrêt rotationnel femelle (15) ménagée sur le bord intérieur du trou (10) de chaque étrier (3, 3a), cette seconde géométrie d'arrêt rotationnel étant adaptée pour venir s'appliquer sur la première géométrie d'arrêt rotationnel après mise en place de l'étrier sur l'axe fileté.

3. Dispositif d'ostéosynthèse rachidienne, comprenant au moins deux éléments d'ancrage osseux (1, 60) dans des corps de la structure osseuse du rachis respectifs (S, L5), au moins un organe (16) de liaison longitudinale entre les éléments d'ancrage osseux, chaque élément d'ancrage osseux comportant une tête (5) de préhension par un outil (6) de vissage, un axe fileté (7) prolongeant la tête de préhension, et un élément de serrage (8) pouvant être mis en place sur cet axe pour bloquer ensemble l'organe (16) de liaison longitudinale et l'élément d'ancrage osseux (1, 60) correspondant, l'axe fileté (7) étant pourvu d'une rotule terminale (11) d'articulation dans un logement (12) d'une calotte sphérique (57) de la tête (5) de préhension, permettant une orientation pluridirectionnelle de l'axe (7) et un positionnement de l'organe (2 ; 16) de liaison longitudinale adaptés à la configuration du segment vertébral (S, L5, ... L2) recevant les éléments d'ancrage osseux, **caractérisé en ce que** l'axe fileté (7) et l'organe (16) de liaison longitudinale sont munis de moyens de blocage (13, 14 ; 38, 41, 43) venant coopérer après introduction de l'axe fileté (7) dans un trou correspondant (38, 41, 43) de passage à travers l'organe (16) de liaison longitudinale, pour bloquer en rotation l'axe et sa rotule (11) par rapport à l'organe (16) de liaison longitudinale.

4. Dispositif selon la revendication 2, **caractérisé en ce qu'**il comprend une plaque (16) ayant des trous (38, 41, 43) dont les bords forment des géométries d'arrêt rotationnel pouvant coopérer avec des géométries d'arrêt rotationnel (13) formées sur une collerette (14) agencée entre la rotule et l'extrémité contiguë de l'axe fileté (7).

5. Dispositif selon l'une des revendications 1 à 4, **caractérisé en ce que** l'extrémité de l'axe fileté (7) opposée à la rotule (11) est constituée d'une forme mâle (21) par exemple en demi-lune, adaptée pour coopérer avec une forme femelle complémentaire (23) d'un outil (6), afin de bloquer la rotule en rotation durant le vissage de l'élément de serrage (8) sur l'axe fileté (7).

6. Dispositif selon l'une quelconque des revendications 1 à 5, **caractérisé en ce que** l'axe fileté (7) est pourvu d'un rétrécissement (18) délimitant deux zones filetées (17) et (19) de cet axe et constituant une amorce de rupture après assemblage et mise en place de l'élément de serrage sur l'étrier de connexion, ce rétrécissement permettant alors la cassure de l'axe (7).

7. Dispositif selon l'une quelconque des revendications 3 à 6, **caractérisé en ce que** l'organe de liaison longitudinale entre les éléments d'ancrage osseux (1, 60) est une plaque (16) dans laquelle sont ménagées des ouvertures cylindriques et/ou oblongues (41, 43) délimitant différents emplacements possibles pour les éléments d'ancrage osseux et traversées par les axes filetés (7), sur lesquels sont mis en place les éléments de serrage (8) de blocage. 1

8. Dispositif selon l'une des revendications 1 à 7, **caractérisé en ce qu'**il comprend un système de liaison transversale entre des éléments d'ancrage osseux (1, 60), formé par une paire de cuvettes (58, 59) chacune monobloc avec une patte (61, 62), la position relative et donc l'écartement entre les cuvettes étant réglable, par exemple au moyen d'un ensemble vis-écrou (63, 64) traversant une lumière allongée (65) d'une patte (61) et un trou taraudé de la deuxième patte (62).

9. Dispositif selon l'une des revendications 1 à 8, **caractérisé en ce que** au moins un élément ancrage osseux (60) est constitué par un crochet lamaire, ce crochet lamaire étant constitué par deux pinces (60a, 60b) à extrémité recourbée et d'écartement relatif réglable.

## Claims

1. A spinal osteosynthesis device, comprising at least two bone anchoring elements (1, 60) for anchoring into respective bodies of the bone structure of the spine (S, L5), at least one longitudinal connecting member (2) between the bone anchoring elements and connecting shackles (3, 3a), each bone anchoring element including a head (5) for grasping by a screwing tool (6), a threaded shank (7) extending the head for grasping and a tightening element (8) which may be mounted on this shank in order to lock together the longitudinal connecting member (2) and the corresponding bone anchoring element (1, 60), the threaded shank (7) being provided with an end ball (11) for a joint in a housing (12) of a spherical cap (57) of the head (5) for grasping allowing multi-directional orientation of the shank (7) and a positioning of the longitudinal connecting member (2) adapted to the configuration of the vertebral segment (S, L5...L2) receiving the bone anchoring elements, **characterized in that** the threaded shank (7) and each connecting shackle (3, 3a) are provided with immobilizing means (13, 14; 15) which will cooperate after introducing the threaded shank (7) into a corresponding through-hole (10) through the connecting shackle (3, 3a), for immobilizing the shank (7) and its ball (11) in terms of rotation relatively to the connecting shackle (3, 3a).

2. The device according to claim 1, **characterized in that** the longitudinal connecting member between the bone anchoring element (1) is a vertebral rod (2) passing through the shackles (3, 3a) for connecting to the bone anchoring elements, and **in that** said immobilizing means comprise at least one rotation-stopping geometry, preferably two of them, i.e. a first rotation-stopping geometry (13) formed on a collar (14) laid out between the ball and the contiguous end of the threaded shank (7) and a second female rotation-stopping geometry (15) provided on the interior edge of the hole (10) of each shackle (3, 3a), this second rotation-stopping geometry being adapted so as to press against the first rotation-stoppinggeometry after mounting the shackle on the threaded shank.

3. The spinal osteosynthesis device, comprising at least two bone anchoring elements (1, 60)) for anchoring into respective bodies of the bone structure of the spine (S, L5), at least one longitudinal connecting member (16) between the bone anchoring elements, each bone anchoring element including a head (5) for grasping with a screwing tool (6), a threaded shank (7) extending the head for grasping, and a tightening element (8) which may be mounted on this shank in order to lock together the longitudinal connecting member (16) and the corresponding bone anchoring element (1, 60), the threaded shank (7) being provided with an end ball (11) for a joint in a housing (12) of a spherical cap (57) of the head (5) for grasping, allowing multi-directional orientation of the shank (7) and a positioning of the longitudinal connecting member (2; 16) adapted to the configuration of the vertebral segment (S, L5,...L2) receiving the bone anchoring element, **characterized in that** the threaded shank (7) and the longitudinal connecting member (16) are provided with immobilizing means (13, 14; 38, 41, 43) which will cooperate after introducing the threaded shank (7) into a corresponding through-hole (38, 41, 43) through the longitudinal connecting member (16), in order to immobilize the shank and its ball (11) in terms of rotation, relatively to the longitudinal connecting member (16).

4. The device according to claim 2, **characterized in that** it comprises a plate (16) with holes (38, 41, 43), the edges of which form rotation-stopping geometries which may cooperate with rotation-stopping geometries (13) formed on a collar (14) laid out between the ball and the contiguous end of the threaded sank (7).

5. The device according to any of claims 1 to 4, **characterized in that** the end of the threaded shank (7) opposite to the ball (11) consists of a male profile (21) for example a half-moon profile, adapted for cooperating with a complementary female profile (23) of a tool (6), in order to immobilize the ball in terms of rotation during the screwing of the tightening element (8) on the threaded shank (7).

6. The device according to any of claims 1 to 5, **characterized in that** the threaded shank (7) is provided with a throat (18) delimiting two threaded areas (17) and (19) of this axis and forming an incipient failure after assembling and mounting the tightening element on the connecting shackle, this throat then allowing the shank (7) to be broken.

7. The device according to any of claims 3 to 6, **characterized in that** the longitudinal connecting member between the bone anchoring elements (1, 60) is a plate (16) in which the cylindrical and/or oblong openings (41, 43) are provided, delimiting various possible locations for the bone anchoring elements and crossed by the threaded axes (7), on which tightening elements (8) are mounted.

8. The device according to any of claims 1 to 7, **characterized in that** it comprises a transverse connecting system between bone anchoring elements (1, 60), formed by a pair of cups (58, 59) each in one piece with a tab (61, 62), the relative position and therefore the distance between the cups being adjustable, for example by means of a screw/nut assembly (63, 64) passing through an elongated lumen (65) of a tab (61) and a tapped hole of the second tab (62).

9. The device according to any of claims 1 to 8, **characterized in that** at least one bone anchoring element (60) is formed by a laminar hook, this laminar hook being formed by two claws (60a, 60b) with a curved end and with adjustable relative separation.

## Patentansprüche

1. Vorrichtung zur Wirbelsäulenosteosynthese, mindestens zwei Knochenverankerungselemente (1, 60) in Körpern der Knochenstruktur der jeweiligen Wirbel (S, L5), mindestens ein Organ (2) zur Längsverbindung zwischen den Knochenverankerungselementen und Bügel zum Verbinden (3, 3a) umfassend, wobei jedes Knochenverankerungselement einen Kopf (5) zum Greifen durch ein Schraubwerkzeug (6), eine Gewindeachse (7), die den Greifkopf verlängert, und ein Spannelement (8), das auf dieser Achse angeordnet werden kann, um die Einheit Längsverbindungsorgan (2) und entsprechendes Knochenverankerungselement (1, 60) zu blockieren, umfasst, wobei die Gewindeachse (7) mit einem Endgelenk (11) zum Anlenken in einer Aufnahme (12) einer kugelförmigen Kalotte (57) des Greifkopfs (5) versehen ist, die eine Mehrrichtungsausrichtung der Achse (7) und ein Positionieren des Längsverbindungsorgans (2) angepasst an die Konfiguration des Wirbelsegments (S, L5, ... L2), das die Knochenverankerungselemente aufnimmt, erlaubt, **dadurch gekennzeichnet, dass** die Gewindeachse (7) und jeder Verbindungsbügel (3, 3a) mit Blockiermitteln (13, 14; 15) ausgestattet sind, die nach dem Einführen der Gewindeachse (7) in eine entsprechende Durchgangsbohrung (10) durch den Verbindungsbügel (3, 3a) zusammenwirken, um die Achse (7) und ihr Gelenk (11) im Verhältnis zum Verbindungsbügel (3, 3a) in Rotation zu blockieren.

2. Vorrichtung nach Anspruch 1, **dadurch gekennzeichnet, dass** das Längsverbindungsorgan zwischen den Knochenverankerungselementen (1) ein Wirbelschaft (2) ist, der die Bügel (3, 3a) zum Verbinden mit den Knochenverankerungselementen durchquert, und **dadurch**, dass die besagten Blockiermittel mindestens eine Rotationsstoppgeometrie aufweisen, vorzugsweise zwei, nämlich eine erste Rotationsstoppgeometrie (13), ausgebildet auf einem Kragen (14), der zwischen dem Gelenk und dem neben der Gewindeachse (7) liegenden Ende eingerichtet ist, und eine zweite Rotationsstoppgeometrie in Buchsenform (15), die auf dem Innenrand der Bohrung (10) jedes Bügels (3, 3a) ausgebildet ist, wobei diese zweite Rotationsstoppgeometrie angepasst ist, um sich nach dem Anbringen des Bügels auf der Gewindeachse auf die erste Rotationsstoppgeometrie zu legen.

3. Vorrichtung zur Wirbelsäulenosteosynthese, mindestens zwei Knochenverankerungselemente (1, 60) in Körpern der Knochenstruktur der jeweiligen Wirbel (S, L5), mindestens ein Organ (16) zur Längsverbindung zwischen den Knochenverankerungselementen umfassend, wobei jedes Knochenverankerungselement einen Kopf (5) zum Greifen durch ein Schraubwerkzeug (6), eine Gewindeachse (7), die den Greifkopf verlängert, und ein Spannelement (8), das auf dieser Achse angeordnet werden kann, um die Einheit Längsverbindungsorgan (2) und entsprechendes Knochenverankerungselement (1, 60) zu blockieren, umfasst, wobei die Gewindeachse (7) mit einem Endgelenk (11) zum Anlenken in einer Aufnahme (12) einer kugelförmigen Kalotte (57) des Greifkopfs (5) versehen ist, was eine Mehrrichtungsausrichtung der Achse (7) und ein Positionieren des Längsverbindungsorgans (2; 16) angepasst an die Konfiguration des Wirbelsegments (S, L5, ... L2), das die Knochenverankerungselemente aufnimmt, erlaubt, **dadurch gekennzeichnet, dass** die Gewindeachse (7) und das Längsverbindungsorgan (16) mit Blockiermitteln (13, 14; 38, 41, 43) ausgestattet sind, die nach dem Einführen der Gewindeachse (7) in eine entsprechende Durchgangsbohrung (38, 41, 43) durch das Längsverbindungsorgan (16) zusammenwirken, um die Achse und ihr Gelenk (11) im Verhältnis zum Längsverbindungsorgan (16) in Rotation zu blockieren.

4. Vorrichtung nach Anspruch 2, **dadurch gekennzeichnet, dass** sie eine Platte (16) mit Bohrungen (38, 41, 43) umfasst, deren Ränder Rotationsstoppgeometrien ausbilden, die imstande sind, mit Rotationsstoppgeometrien (13) zusammenzuwirken, die auf einem Kragen (14) ausgebildet sind, der zwischen dem Gelenk und dem benachbarten Ende der Gewindeachse (7) ausgebildet ist.

5. Vorrichtung nach einem der Ansprüche 1 bis 4, **dadurch gekennzeichnet, dass** das dem Gelenk (11) gegenüberliegende Ende der Gewindeachse (7) von einer zum Beispiel halbmondförmigen Steckform (21) gebildet wird, die angepasst ist, um mit einer komplementären Buchsenform (23) eines Werkzeugs (6) zusammenzuwirken, um das Gelenk bei der Verschraubung des Spannelement (8) auf der Gewindeachse (7) in Rotation zu blockieren.

6. Vorrichtung nach einem der Ansprüche 1 bis 5, **dadurch gekennzeichnet, dass** die Gewindeachse (7) mit einer Verjüngung (18) versehen ist, die zwei Gewindezonen (17) und (19) dieser Achse begrenzt und nach Montage und Platzierung des Spannelements auf dem Verbindungsbügel einen Bruchansatz bildet, wobei diese Verjüngung dann das Brechen der Achse (7) erlaubt.

7. Vorrichtung nach einem der Ansprüche 3 bis 6, **dadurch gekennzeichnet, dass** das Längsverbindungsorgan zwischen den Knochenverankerungselementen (1, 60) eine Platte (16) ist, in die zylindrische und/oder längliche Öffnungen (41, 43) eingearbeitet sind, die verschiedene mögliche Stellen für die Knochenverankerungselemente und Durchführungen für die Gewindeachsen (7) begrenzen, auf denen die blockierenden Spannelemente (8) platziert werden.

8. Vorrichtung nach einem der Ansprüche 1 bis 7, **dadurch gekennzeichnet, dass** sie ein System zur Querverbindung zwischen den Knochenverankerungselementen (1, 60), gebildet aus einem Paar Schalen (58, 59) jeweils aus einem Stück mit einer Pratze (61, 62) umfasst, wobei die relative Stellung und daher der Abstand zwischen den Schalen einstellbar ist, zum Beispiel mittels einer Schrauben-Mutter-Einheit (63, 64), die ein Langloch (65) einer Pratze (61) und eine Gewindebohrung der zweiten Pratze (62) durchquert.

9. Vorrichtung nach einem der Ansprüche 1 bis 8, **dadurch gekennzeichnet, dass** mindestens ein Knochenverankerungselement (60) von einem Klingenhaken gebildet wird, wobei dieser Klingenhaken von zwei Zangen (60a, 60b) mit zurückgebogenem Ende und verstellbarer relativer Beabstandung gebildet wird.
